# EUROPEAN PATENT APPLICATION

(11) **EP 1 738 802 A1**
(43) Date of publication of application: **03.01.2007**
(21) Application number: 06012657.0
(22) Date of filing: 20.06.2006
(51) Int. Cl.: A61Q 11/00, A61K 8/81, A61K 8/38

(54) **Pasty dental bleaching material**

(30) Priority: 30.06.2005 JP 2005192485
(71) Applicant: GC Corporation, Tokyo 174-8585 (JP)
(72) Inventor: Mori, Daizaburo, Itabashi-ku Tokyo 174-8585 (JP); Yamaguchi, Shin, Itabashi-ku Tokyo 174-8585 (JP); Ikushima, Keisuke, Itabashi-ku Tokyo 174-8585 (JP)
(74) Representative: Müller-Boré & Partner Patentanwälte

(57) **Abstract**

An objective of the present invention is to provide a pasty dental bleaching material, which can stably preserve hydrogen peroxide for a long period of time, has excellent adhesion with viscosity being about same level as that of the conventional material, gives few damages to a tooth by pH, and has high efficiency of bleaching to tooth. The pasty dental bleaching agent in the present invention has two structures. In one structure, 10-50 wt.% powdery hydrogen peroxide polyvinyl pyrrolidone complex is blended in a liquid component. In another structure, the pasty dental bleaching agent comprises a pasty first component and a pasty second component. In the first component, 10-50 wt.% powdery hydrogen peroxide polyvinyl pyrrolidone complex is blended in a liquid component. In the second component, 0.01-10 wt.% catalyst for activating hydrogen peroxide and a 1-70 wt.% thickener are blended in a liquid component.

## Description

The present invention relates to a dental bleaching material for bleaching a tooth, which is discolored by depositing pigment, by using hydrogen peroxide, and more particularly, relates to a pasty dental bleaching material, which can stably preserve hydrogen peroxide for a long period of time, has excellent adhesion with viscosity being about same level as that of the conventional material, gives few damages to the tooth by pH, and has high efficiency in bleaching of tooth.

It is generally thought that dental whiteness is an important element of beauty. Further, it is strongly desired for young women to make a tooth white, and requirement for a dental bleaching has been increased. As a method of the dental bleaching, that is, dental whitening, a method by using hydrogen peroxide or titanium oxide has been mainly used.

As a method by using hydrogen peroxide, for example, the following three methods are known. One method is applying a bleaching agent on the surface of a vital tooth, in which the agent is made by mixing 35% hydrogen peroxide solution with silicic anhydride (refer to Japanese Patent Application Laid Open No. 5(1993)-320033). The other method is applying a bleaching agent to the surface of a tooth, in which the agent is made by mixing 35% hydrogen peroxide solution and orthophosphoric acid, and further mixing them, hydroxyl apatite and fluoride if necessary (refer to Japanese Patent Application Laid Open No. 8(1996)-143436). The other method is putting a bleaching agent in a dental tray so as to contact with a tooth to be bleached, in which the bleaching agent is made by dispersing carbamideperoxide in a matrix material containing carboxymethylene (refer to Japanese Patent Application Laid Open No. 8(1996)- 113520).

However, hydrogen peroxide solution is remarkably unstable, and easily decomposed by change with passage of time or preservation condition, so that an objective bleaching effect cannot be exercised in many cases. Further, the following means has been used so that the adhesion to the tooth surface is enhanced, and operativity is enhanced in order not to easily flow and remove a composition. That means is adding a thickener to a liquid containing hydrogen peroxide to thereby increase the viscosity of the liquid, in which the thicker is carboxypolymethylene, polyvinylpyrrolidone, hydroxyethylcellulose or hydroxymethylcelluloses, saponite or magnesium sodium lithium silicate or the like. However, when the thickener and hydrogen peroxide exist in the same liquid, there is a problem that both activities of the thickener and hydrogen peroxide are reduced. Thickening action is also reduced by the reduction of activity of hydrogen peroxide, so that there is a problem that viscosity of the dental bleaching material is remarkably reduced.

Further, in order to efficiently bleach the tooth, it is desired that the dental bleaching material acting to the tooth surface has pH being from nearly neutral to alkalescence. However, in the conventional method by using hydrogen peroxide, pH which peroxide inherently has is 5.0 or less, and pH of the dental bleaching material is adjusted to acidity in order to enhance stability of hydrogen peroxide. When bleaching is carried out with such a low pH, there are problems that the tooth surface is decalcified to thereby damage dentin.

In order to avoid the above-described problem, it is also considered that the dental bleaching material is adjusted on the alkali side beforehand, and this idea is desired to reduce damaging of the tooth and from a point of bleaching efficiency. However, if the pH of the dental bleaching material is adjusted to alkalinity, preservation stability of hydrogen peroxide which is an effective component is remarkably reduced, so that it was not realized from a point of a stable product supply to a user.

An objective of the present invention is to provide a dental bleaching material using hydrogen peroxide, which can be stably preserved for a long period of time, has excellent adhesion with viscosity being about same level as that of the conventional material, gives few damages to the tooth by pH, and has high efficiency in bleaching of tooth.

The earnest work was carried out in order to solve the above-mentioned problems and, as a result of this, the followings were found out to complete the present invention. In the dental bleaching material using hydrogen peroxide, the conventional thickeners are not blended with hydrogen peroxide, so that decompressing of hydrogen peroxide with the thickener can be prevented at the time of the preservation. Further, when a hydrogen peroxide polyvinylpyrrolidone complex is used as a new thickener and bleaching component, the viscosity of the composition can be stably obtained for a long time of period while hydrogen peroxide being stably preserved. Furthermore, it is not necessary to adjust the dental bleaching material to acidity in order to stabilize hydrogen peroxide as in the conventional method, so that damaging to the tooth by pH can be prevented.

That is, one aspect of the present invention is to provide a pasty dental bleaching material in which 10-50 wt.% powdery hydrogen peroxide polyvinylpyrrolidone complex is blended in a liquid component. The liquid component, which can be preferably used, is at least one kind selected from water, ethanol, 1- propanol, 2- propanol, 2- methyl-2- propanol, glycerine, diglycerine, polyglycerine, propyleneglycol, dipropyleneglycol, polypropyleneglycol, ethyleneglycol, diethyleneglycol, polyethyleneglycol, polyethyleneglycolmonomethylether, 1, 2 pentanediol, 1, 2 hexanediol, and 1, 2 octanediol. Further, the pasty dental bleaching material is preferably blended with 5-30 wt.% hydrogen peroxide, and blended with a pH regulator to have the pH of 6 to 8.

Another aspect of the present invention is to provide a pasty dental bleaching material comprising a pasty first component and a pasty second component. The first component is made by blending 10-50 wt.% powdery hydrogen peroxide polyvinylpyrrolidone complex in a liquid component, and the second component is made by blending 0.01-10 wt . % catalyst for activating hydrogen peroxide and 1-70 wt.% thickener in a liquid component. The liquid component, which can be preferably used in the first and second components, is at least one kind selected from water, ethanol, 1-propanol, 2- propanol, 2- methyl- 2- propanol, glycerine, diglycerine, polyglycerine, propyleneglycol, dipropyleneglycol, polypropyleneglycol, ethyleneglycol, diethyleneglycol, polyethyleneglycol, polyethyleneglycolmonomethylether, 1, 2 pentanediol, 1, 2 hexanediol, and 1, 2 octanediol. Further, the first component is preferably blended with 5-30 wt.% hydrogen peroxide, and a pH regulator is blended in the first and/or second components to have pH of 6 to 8 when the first and second components are mixed at a desired ratio.

In the pasty dental bleaching material according to the present invention, the predetermined amount of the powdery hydrogen peroxide polyvinylpyrrolidone complex is blended in the liquid component, in order to solve the problem of the conventional pasty bleaching material using hydrogen peroxide that hydrogen peroxide cannot be stably preserved for a long time of period. Accordingly, it is possible to make the pasty dental bleaching material, which can stably preserve hydrogen peroxide for a long period of time, has excellent adhesion with viscosity being about same level as that of the conventional material, gives few damages to the tooth by pH, and has high efficiency in bleaching of tooth.

One aspect of the pasty dental bleaching material according to the present invention is that 10-50 wt.% powdery hydrogen peroxide polyvinylpyrrolidone complex is blended in the liquid component. The liquid component is to dissolve hydrogen peroxide polyvinylpyrrolidone complex. The liquid component is not limited especially, if it is safe to an organism and is a component which does not react to or decompose hydrogen peroxide polyvinylpyrrolidone complex. However, when it is considered that the liquid component is used in an oral cavity, water or a water-soluble solvent such as alcohols or the like is preferable.

More particularly, the liquid component, which can be preferably used, is water, ethanol, 1- propanol, 2- propanol, 2- methyl- 2- propanol, glycerine, diglycerine, polyglycerine, propyleneglycol, dipropyleneglycol, polypropyleneglycol, ethyleneglycol, diethyleneglycol, polyethyleneglycol, polyethyleneglycolmonomethylether, 1, 2 pentanediol, 1, 2 hexanediol, and 1, 2 octanediol.

The hydrogen peroxide polyvinylpyrrolidone complex used in the pasty dental bleaching material according to the present invention is a powdery complex of polyvinylpyrrolidone containing hydrogen peroxide, which is indicated, for example, in Japanese translation of PCT international application No. 6(1994)- 505765. It is preferable that concentration of hydrogen peroxide in the complex is 1 to 30 wt.%. The hydrogen peroxide polyvinylpyrrolidone complex has a feature that hydrogen peroxide can be stably preserved for a long time of period in a solution or even under a severe condition, i.e. alkali or the like, and polyvinylpyrrolidone itself is not influenced by hydrogen peroxide for a long time of period. Further, the hydrogen peroxide polyvinylpyrrolidone complex keeps the property of polyvinylpyrrolidone, has an excellent thickening action with respect to the composition to be blended, and is safe to an organism.

It is necessary that a blending amount of the powdery hydrogen peroxide polyvinylpyrrolidone complex is 10 to 50 wt.%. If the blending amount is less than 10 wt . % , the viscosity of the pasty composition is too low, so that the operativity is insufficient. Further, if the blending amount is more than 50 wt.%, the viscosity of the pasty dental bleaching material is too high, so that the operativity is insufficient. More preferably, the blending amount is 25 to 50 wt.%.

In the pasty dental bleaching material according to the present invention, if 5-30 wt. % hydrogen peroxide is further blended, the efficiency of bleaching can be enhanced, so that it is preferable. It is more preferable that the blending amount of hydrogen peroxide is 10 to 30 wt.%.

Another aspect of the present invention is to provide the pasty dental bleaching material comprising the first component comprising the above-described pasty dental bleaching material, and a pasty second component made by blending 0.01-10 wt.% catalyst for activating hydrogen peroxide and 1-70wt.% thickener in the liquid component. When 5-30 wt.% hydrogen peroxide is further blended in the first component, the pH regulator is blended in the first and/or second components so as to have pH of 6 to 8 when the first and second component are mixed at the desired ratio.

As the pH regulator, disodium phosphate, potassium hydroxide, sodium hydroxide, sodium carbonate, triethanolamine or the like is preferably used.

The second component of the pasty dental bleaching material according to the present invention comprising the two components is mixed with the first component at the desired ratio before using it, for activating hydrogen peroxide of hydrogen peroxide polyvinylpyrrolidone complex, and also activating hydrogen peroxide when 5-30 wt.% hydrogen peroxide is further blended in the first component. Therefore, when hydrogen peroxide is blended in the pasty dental bleaching material according the present invention comprising the two components, it is necessary to blend hydrogen peroxide only in the first component. The catalyst for activating hydrogen peroxide blended in the second component is stable in the second component, and has an operation for activating hydrogen peroxide in the first component only when it is mixed with the first component.

As the catalyst, more particularly, titanium dioxide, titanium nitroxide, manganese dioxide, an iron compound, a copper compound or the like can be used. In these catalysts, titanium nitroxide is the most suitable catalyst since hydrogen peroxide can be most-suitably activated by this catalyst with a wavelength of a photopolymerizer mostly used in dentistry.

The blending amount of the catalyst for activating hydrogen peroxide used in the pasty dental bleaching material according to the present invention is 0.01 to 10 wt.% in the second component. If the amount is less than 0.01 wt.%, the operation for activating hydrogen peroxide is insufficient when mixing with the first component. If the amount is more than 10 wt.%, the activation of hydrogen peroxide after mixing is advanced too quickly, so that the bleaching effect when using the material is reduced. More preferably, the blending amount of the catalyst is 0.5 to 5 wt.%.

In the second component of the pasty dental bleaching material according to the present invention, it is necessary to blend 1-70 wt . % thickener in order to enhance operativity in a pasty state. As the thickener, it is possible to use a thickener which is conventionally used for the dental bleaching material and suitably used in an oral cavity, such as an organic thickener or an inorganic thickener. The organic thickener is, for example, sodium cellulose glycolate, sodium alginate, a carboxymethyl cellulose, a sodium carboxymethyl cellulose, a calcium carboxymethyl cellulose, carboxypolymethylene, a methylvinylether / maleic anhydride copolymer, acrylic acid / behenic acid copolymer,dimethylpolysiloxane, sodium starch glycolate, sodium starch phosphate, sodium polyacrylate, a methyl cellulose, a crystalline cellulose, a hydroxylethyl cellulose, ahydroxylmethyl cellulose, a hydroxylpropyl cellulose, a polyvinyl pyrrolidone or the like. The inorganic thickener is, for example, magnesium sodium silicate, magnesium sodium lithium silicate, calcium carbonate, calcium silicate, magnesium silicate, a silica powder, various glasses, an amorphous hydrous silicic acid, a fumed silica or the like.

As for the blending amount of the thickener blended in the second component, if it is less than 1 wt.%, a thickening effect is hardly obtained, and if it is more than 70 wt . %, the viscosity of the second component is too high, so that the operativity may be reduced. More preferably, the blending amount of the thickener is 3 to 40 wt.%.

In the pasty dental bleaching material according to the present invention, a component such as a perfume, a pigment, a stabilizer, a solvent or the like can be blended to either component within a range in which the effects of the components used in the present invention are not prevented.

As for a method for bleaching a discolored tooth by using the pasty dental bleaching material according to the present invention, when the pasty dental bleaching material according to the present invention comprises only the one component, a method of coating the pasty dental bleaching material on the surface of the discolored tooth, or a method of taking the pasty dental bleaching material into a specified tray and adhering it on the tooth is generally used.

Further, as for a method for bleaching the discolored tooth by using the pasty dental bleaching material according to the present invention comprising the first component and the second component, a method comprising the steps of mixing the two components at the desired ratio just before using those; and coating the mixture on the tooth surface; or taking it into the specified tray and adhering it on the tooth is used.

As for a bleaching time of the tooth, in the both cases of the pasty dental bleaching material according to the present invention comprising only the one component and comprising the two components, it takes several minutes to several hours. Further, after applying the pasty dental bleaching material according to the present invention to the tooth surface in this way, the same operation is repeated if necessary with intervals until the effect can be obtained. Further, the pasty dental bleaching material according to the present invention has the high bleaching effect by irradiation of light. More particularly, in the case of the dental bleaching material comprising the two components, when the light is irradiated by a light irradiator after applying the material to the tooth, the catalyst is more activated to thereby enhance the bleaching effect, so that it is preferable.

### [Example]

As shown in examples in Tables 1, a pasty dental bleaching material comprising only one component was produced by the steps of stirring one or more kinds of water, ethanol, glycerine and polyethylene glycol (a weight average molecular weight: 600) as the liquid component; adding to it a hydrogen peroxide polyvinyl pyrrolidone complex, containing 20 wt.% hydrogen peroxide and having a weight average molecular weight of 58,000 (a product name: Peroxydone K-30 produced by ISP Corporation), or a hydrogen peroxide polyvinyl pyrrolidone complex containing 20 wt.% hydrogen peroxide and having a weight average molecular weight of 1,300,000 (a product name: Peroxydone K-90 produced by ISP Corporation) while stirring those; and adding hydrogen peroxide and disodium phosphate or potassium hydroxide, as a pH regulator if necessary, while stirring those.

Further, as shown in examples in Tables 2 and 3, a first component and a second component of the pasty dental bleaching material comprising the two components was produced by the steps of stirring one or more kinds of water, polyethylene glycol (a weight average molecular weight: 400) and glycerine as the liquid component; adding titanium nitroxide (a product name: V-CAT, produced by Toyota Tsusho Corporation) or manganese dioxide (produced by Wako Pure Chemical Corporation) as the catalyst for activating hydrogen peroxide, while stirring those; adding a polyvinyl pyrrolidone, dimethylpolysiloxane, sodium magnesium silicate, a methylethylhydroxylethyl cellulose or a methylvinylether / maleic anhydride copolymer as the thickener, while stirring those; and adding sodium carbonate, sodium hydroxide, disodium phosphate, triethanolamine or potassium hydroxide as the pH regulator, if necessary, while stirring those.

Further, as shown in comparison examples in Table 4, pasty dental bleaching material comprising only one component was produced by the steps of stirring hydrogen peroxide and water, and if necessary further the polyethylene glycol (a weight average molecular weight: 600) as the liquid component; adding a polyvinyl pyrrolidone, dimethylpolysiloxane, sodium magnesium silicate, a methylethylhydroxylethyl cellulose or a methylvinylether / maleic anhydride copolymer as the thickener, while stirring those; and adding potassium hydroxide as the pH regulator, if necessary, while stirring those.

### <Evaluation of concentration lowering rate of hydrogen peroxide>

In order to evaluate how the concentration of hydrogen peroxide of the paste blended with hydrogen peroxide was changed by passing time, the concentration decreasing rate of hydrogen peroxide was measured and evaluated by the following method after two weeks from the producion time.

This evaluation was carried out by a titration method using potassium iodide and ammonium molybdate.
1) 0.1 g of a sample was mixed into an aqueous solution comprising 25 mL of distilled water and 5 mL of glacial acetic acid.
2) 2 g of the potassium iodide and one drop of the ammonium molybdate were added to the solution and the sample solution was titrated by 0 . 1 mol/L of sodium thiosulfate after leaving it.
3) 3 mL of a starch aqueous solution was added, and an amount of the sodium thiosulfate was measured when the color of the liquid was changed from dark blue to colorlessness.
   In addition, the amount was calculated using a conversion that 1 mL of 0.1N sodium thiosulfate is equal to 1.701 mg of hydrogen peroxide.
4) The sample was filled in a syringe made of polypropylene to be kept at 60°C for two weeks.
5) After the sample being kept, it was re-measured by the methods from 1) to 3).
6) The decreasing rate of the concentration of hydrogen peroxide was calculated with the following formula. The formula is, [1- (Concentration after keeping /Concentration before keeping)×100 Evaluation

○ : The decreasing rate of hydrogen peroxide was less than 5 wt.%.
Δ : The decreasing rate of hydrogen peroxide was from 5 wt.% or more to less than 10 wt.%.
× : The decreasing rate of hydrogen peroxide was 10 wt . % or more.

### <Evaluation of a gel state (evaluation of stability of the thickener)>

In order to evaluate how the gel state of the paste blended with hydrogen peroxide was changed especially by a passing of time of the thickener, the gel state was measured and evaluated by the following method after two weeks from the producion time.
1) 0.5 mL of the sample was measured and taken on a glass kneading board, and kept in a thermostatic device at 37°C for 5 minutes.
2) Sizes of a maximum portion and a minimum portion between parallel cutting lines of the expansion of the sample, and an average value of those was obtained.
3) The sample was kept at 60°C for two weeks, and it was measured like the above-described method.
4) The gel state was evaluated from the difference of the expansions of the samples before and after keeping. Evaluation

○: The difference was less than 2 mm.
Δ: The difference was less than 4 mm.
×: The difference was 4 mm or more.

### <Evaluation of the pH value at the time of use>

In the case of the pasty dental bleaching material comprising only one component, the pH in the state after two weeks passing from the production time was measured. In the case of the pasty dental bleaching material comprising two components, the pH just after mixing the two pastes after two weeks passing from the production time was measured. Those pH were measured by the following method.
1) 5 g of the dental bleaching material were taken on a bottom of a beaker by a spatula.
2) 5 g of distilled water was added, and the water was flowed out lightly after 10 minutes and then, the pH of a part of the dental bleaching material was measured. Evaluation

○ : The pH was from 6.5 to 7.5.
Δ : The pH was 6.0 or more but less than 6.5.
X: The pH was less than 6.0

Results of the evaluation of the concentration decreasing rate of hydrogen peroxide, the evaluation of the gel state (the evaluation of the stability of the thickener), and the evaluation of the pH value at the time of use were collectively shown in Tables 1, 2, 3 and 4.

**Table 1**

| [Unit: wt.%] | | | | | | | | | |
|---|---|---|---|---|---|---|---|---|---|
| | | Example 1 | Example 2 | Example 3 | Example 4 | Example 5 | Example 6 | Example 7 | Example 8 |
| Dental Bleaching Material comprising only one component | Hydrogen Peroxide Polyvinyl Pyrrolidone Complex | Peroxydone K-90 | Peroxydon K-30 | Peroxydone K-90 | Peroxydone K-90 | Peroxydone K-30 | Peroxydone K-90 | Peroxydone K-30 | Peroxydone K-90 |
| | | 45 | 15 | 30 | 25 | 25 | 15 | 20 | 30 |
| | Liquid Component | Ethanol | Glycerine | Polyethylene Glycol | Hydrogen Peorxide | Hydrogen Peorxide | Hydrogen Peorxide | Hydrogen Peorxide | Hydrogen Peorxide |
| | | 55 | 55 | 5 | 25 | 22 | 20 | 10 | 10 |
| | | | Water | Glycerine | Water | Water | Water | Polyethylene Glycol | Glycerine |
| | | | 30 | 50 | 45 | 47 | 64.9 | 16 | 46 |
| | | | | Water | | | | Water | Water |
| | | | | 15 | | | | 53.98 | 13 |
| | pH Regulator | - | - | - | Disodium Phosphate | Disodium Phosphate | Potassium Hydroxide | Potassium Hydroxide | Disodium Phosphate |
| | | | | | 5 | 6 | 0.1 | 0.02 | 1 |
| Concentration Decreasing Rate of Hydrogen Peroxide | | ○ | ○ | ○ | ○ | Δ | Δ | ○ | ○ |
| Gel State | | ○ | ○ | ○ | ○ | ○ | ○ | ○ | ○ |
| pH | | ○ | ○ | ○ | ○ | ○ | ○ | ○ | ○ |

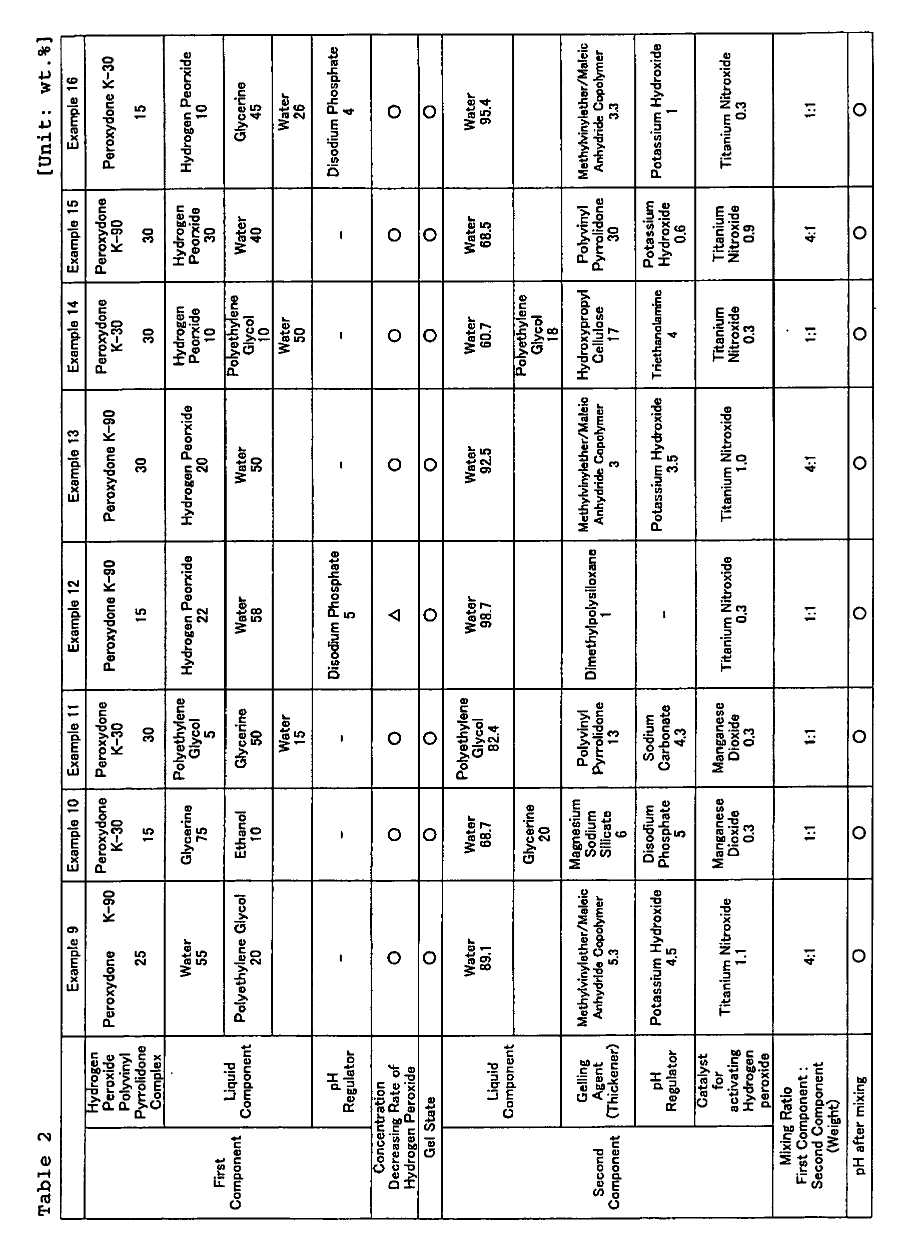

**Table 3**

| [Unit: wt.%] | | | | | | | | |
|---|---|---|---|---|---|---|---|---|
| | | Example 17 | Example 18 | Example 19 | Example 20 | Example 21 | Example 22 | Example 23 |
| First Component | Hydrogen Peroxide Polyvinyl Pyrrolidone Complex | Peroxydone K-30 | Peroxydone K-30 | Peroxydone K-90 | Peroxydone K-90 | Peroxydona K-30 | Peroxydons K-90 | Peroxydone K-30 |
| | | 20 | 30 | 30 | 20 | 30 | 30 | 30 |
| | Liquid Component | Water | Water | Water | Water | Hydrogen Peorxide | Hydrogen Peorxide | Hydrogen Peorxide |
| | | 80 | 66.5 | 70 | 80 | 15 | 15 | 15 |
| | | | | | | Water 55 | Water 55 | Water 54.96 |
| | pH Regulator | - | Potassium Hydroxide 3.5 | - | - | - | - | Potassium Hydroxide 0.04 |
| Concentration Decreasing Rate of Hydrogen Peroxide | | ○ | ○ | ○ | ○ | ○ | ○ | ○ |
| Jelly State | | ○ | ○ | ○ | ○ | ○ | ○ | ○ |
| Second Component | Liquid Component | Water 66 | Water 86.6 | Water 74.7 | Water 90.3 | Water 83.4 | Water 49 | Water 91.7 |
| | | Polyethylene Glycol 20 | | | | | Polyethylene Glycol 30 | |
| | Gelling Agent (Thickener) | Dimethylpolysiloxane 10 | Polyvinyl Pyrrolidone 13 | Methylvinylether/Maleic Anhydride Copolymer 13 | Magnesium Sodium Silicate 9 | Polyvinyl Pyrrolidone 13 | Methylvinylether/Maleic Anhydride Copolymer 10 | Methylethylhydroxylethyl Cellulose 8 |
| | pH Regulator | Potassium Hydroxide 3.5 | | Potassium Hydroxide 8.3 | - | Potassium Hydroxide 3.5 | Potassium Hydroxide 8 | - |
| | Catalyst for activating Hydrogen peoxide | Titanium Nitroxide 0.5 | Titanium Nitroxide 0.4 | Manganese Dioxide 4 | Titanium Nitroxide 0.7 | Titanium Nitroxide 0.1 | Titanium Nitroxide 3 | Titanium Nitroxide 0.3 |
| Mixing Ratio First Component Second Component (Weight) | | 1:1 | 1:1 | 5:1 | 1:1 | 1:1 | 4:1 | 1:1 |
| pH after mixing | | ○ | ○ | ○ | ○ | ○ | ○ | ○ |

**Table 4**

| [Unit: wt.%] | | | | | | | | |
|---|---|---|---|---|---|---|---|---|
| | | Comparison example 1 | Comparison example 2 | Comparison example 3 | Comparison example 4 | Comparison example 5 | Comparison example 6 | Comparison example 7 |
| | Gelling Agent (Thickener) | Polyvinyl Pyrrolidone 15 | Polyvinyl Pyrrolidone 15 | Polyvinyl Pyrrolidone 30 | Dimethylpolysiloxane 30 | Sodium Magnesium Silicate 20 | Methylethylhydroxylethyl Cellulose 30 | Methylvinylether/Maleic Anhydride Copolymer 15 |
| | Liquid Component | Hydrogen Peorxide 24.5 | Hydrogen Peorxide 24.5 | Hydrogen Peorxide 30 | Hydrogen Peorxide 24.5 | Hydrogen Peorxide 30 | Hydrogen Peorxide 20.5 | Hydrogen Peorxide 24.5 |
| | | Water 45.5 | Water 42 | Water 40 | Water 45.5 | Water 40 | Water 45.5 | Water 45.5 |
| | | Polyethylene Glycol 15 | Polyethylene Glycol 15 | | | Polyethylene Glycol 10 | | Polyethylene Glycol 15 |
| | pH Regulator | - | Potassium Hydroxide 3.5 | - | - | - | Potassium Hydroxide 4 | - |
| Concentration Decreasing Rate of Hydrogen Peroxide | | × | × | × | × | × | × | × |
| Gel State | | × | Δ | × | × | × | Δ | × |
| pH | | × | Δ | × | × | × | Δ | × |

As for the pasty dental bleaching material according to the present invention, it can be confirmed from Tables 1, 2, 3 and 4 that the value of the concentration decreasing rate of hydrogen peroxide was low and the evaluation of the gel state (the evaluation of the stability of the thickener) was sufficient and, further, the pH value at the time of use was near to neutrality, so that there was little damage to the tooth by the pH.

## Claims

1. A pasty dental bleaching material,
wherein 10-50 wt.% powdery hydrogen peroxide polyvinyl pyrrolidone complex is blended in a liquid component.

2. The pasty dental bleaching material according to claim 1,
wherein the liquid component is at least one kind selected from water, ethanol, 1- propanol, 2- propanol, 2- methyl- 2- propanol, glycerine, diglycerine, polyglycerine, propyleneglycol, dipropyleneglycol, polypropyleneglycol, ethyleneglycol, diethyleneglycol, polyethyleneglycol, polyethyleneglycolmonomethylether, 1, 2 pentanediol, 1, 2 hexanediol, and 1, 2 octanediol.

3. The pasty dental bleaching material according to claim 1 or 2,
wherein 5-30 wt.% hydrogen peroxide is further blended, and a pH regulator is blended so as to have the pH of 6 to 8.

4. A pasty dental bleaching material comprising a pasty first component and a pasty second component,
wherein the first component is made by blending 10-50 wt.% powdery hydrogen peroxide polyvinyl pyrrolidone complex in a liquid component, and the second component is made by blending 0.01-10 wt.% catalyst for activating hydrogen peroxide and 1-70 wt.% thickener in a liquid component.

5. The pasty dental bleaching material according to claim 4,
wherein the liquid components of the first and second components are at least one kind selected from water, ethanol, 1- propanol, 2- propanol, 2- methyl- 2-propanol, glycerine, diglycerine, polyglycerine, propyleneglycol, dipropyleneglycol, polypropyleneglycol, ethyleneglycol, diethyleneglycol, polyethyleneglycol, polyethyleneglycolmonomethylether, 1, 2 pentanediol, 1, 2 hexanediol, and 1, 2 octanediol.

6. The pasty dental bleaching material according to claim 4 or 5,
wherein 5-30 wt.% hydrogen peroxide is further blended in the first component, and a pH regulator is blended in the first component and the second component so as to have the pH of 6 to 8 when the first component and the second component are mixed at a desired ratio.
